# EUROPEAN PATENT APPLICATION

(11) **EP 1 343 008 A1**
(43) Date of publication of application: **10.09.2003**
(21) Application number: 01981039.9
(22) Date of filing: 09.11.2001
(51) Int. Cl.: G01N 33/53, G01N 33/566, G01N 37/00, C12M 1/00, C12N 15/09

(54) **SUPPORTS FOR HYBRIDIZATION AND METHOD OF IMMOBILIZING HYBRID**

(30) Priority: 13.11.2000 JP 2000344651
(71) Applicant: Toyo Kohan Co., Ltd., Tokyo 102-8447 (JP)
(72) Inventor: Okamura, Hiroshi, c/o Toyo Kohan Co. Ltd, Kudamatsu-shi, Yamaguchi 744-8611 (JP); Tanga, Michifumi, c/o Toyo Kohan Co. Ltd, Kudamatsu-shi, Yamaguchi 744-8611 (JP); Yamakawa, Kaoru, c/o Toyo Kohan Co. Ltd, Kudamatsu-shi, Yamaguchi 744-8611 (JP); Ohba, Mitsuyoshi, c/o Toyo Kohan Co. Ltd, Kudamatsu-shi, Yamaguchi 744-8611 (JP); Takagi, Kenichi, c/o Toyo Kohan Co. Ltd, Kudamatsu-shi, Yamaguchi 744-8611 (JP)
(74) Representative: VOSSIUS & PARTNER
(86) International application number: JP0109798
(87) International publication number: WO02039111

(57) **Abstract**

It is intended to provide supports for hybridization useful in the fields of molecular biology, biochemistry and the like whereby DNA can be efficiently clarified without injuring the terminal parts of the DNA. A support having an oligonucleotide, a cDNA or a gDNA immobilized thereon which is produced by chemically modifying a support, immobilizing the oligonucleotide, cDNA or gDNA hybridized therewith and then dehybridizing it to thereby give a support for immobilizing a nucleotide having an oligonucleotide boded thereto.

## Description

### TECHNICAL FIELD

The present invention relates to a support for hybridization capable of immobilizing nucleic acid thereon and useful in the field of molecular biology and gene engineering-related technology, and to a method of immobilizing a hybrid of an oligonucleotide, cDNA or gDNA.

### BACKGROUND ART

Gene analysis is useful in the field of molecular biology and gene engineering technology, and these days it is utilized even in the field of medicine for detecting diseases, etc.

For gene analysis, DNA chips have been developed recently to significantly accelerate the analysis speed. However, the conventional DNA chips are prepared by applying a polymer such as polylysine onto the surface of a glass slide or a silicon substrate followed by immobilizing a DNA thereon. Apart from it, also employed is a method of synthesizing an oligonucleotide on a glass substrate according to semiconductor technology such as photolithography.

However, in the method of applying a polymer such as polylysine onto the surface of a glass slide or a silicon substrate for immobilizing a DNA thereon, the DNA immobilization is unstable, and the method is problematic in that the DNA peels off in a hybridization step or a washing step. In addition, the DNA chips fabricated according to semiconductor technology are problematic in that they are extremely expensive since the production process for them is complicated.

To solve the problems, a DNA must be densely and firmly immobilized on the surface of a solid support.

On the other hand, known is a solid support of which the surface has been chemically modified. However, when a desired DNA is directly bonded to the chemically-modified part via amido-bonding, the terminal parts of the DNA may be damaged through treatment in chemical bonding or the like. Therefore, it is desired to improve it.

An object of the present invention is to provide a support for nucleotide immobilization, which enables efficient DNA analysis not injuring the terminal parts of DNA as in the above and which is useful in the field of molecular biology, gene engineering technology, etc.

### DISCLOSURE OF THE INVENTION

The present inventors have found that, when a hybridized oligonucleotide is immobilized on a chemically-modified support for immobilization, then the oligonucleotide is immobilized in the direction vertical to the surface of the immobilization support and makes it easy to read the DNA information of monobasic polymorphism.

Specifically, the invention of claim 1 provides a support for hybridization, which is characterized in that a hybridized oligonucleotide, cDNA or gDNA is immobilized thereon. Preferably, the hybridized oligonucleotide, cDNA or gDNA contains at least two continuous, primary amine-having nucleotides. Also preferably, the primary amine-having nucleotide is deoxyadenylic acid, deoxycytidylic acid or deoxyguanylic acid. Also preferably, the primary amine-having nucleotides exist on the side of the 5'-terminal. Also preferably, the oligonucleotide has a 5'-overhang terminal. The cDNA may be synthesized by the use of an oligonucleotide, as a primer, which contains at least two continuous, primary amine-having nucleotides on the side of the 5'-terminal. The gDNA is characterized in that it is cleaved with a restriction element so as to contain at least two continuous, primary amine-having nucleotides on the side of the 5'-terminal. The gDNA is characterized in that it is cleaved with a restriction element so as to contain at least two continuous, primary amine-having nucleotides on the side of the 5'-overhang terminal. Further, it is desirable that deoxyadenylic acid, deoxycytidylic acid or deoxyguanylic acid is immobilized on the side of the support for hybridization. Also preferably, the hybridized oligonucleotide, cDNA or gDNA has from 1 to 10 primary amine-having nucleotides at the terminal for immobilization.

The invention of claim 11 is a method of immobilizing a hybrid of an oligonucleotide, cDNA or gDNA, which comprises chemically modifying a support for hybridization, immobilizing a hybridized oligonucleotide, cDNA or gDNA on it, and then dehybridizing the complementary oligonucleotide, cDNA or gDNA not immobilized on the support. Preferably, the chemical modification of the support for hybridization comprises chlorination, amination and carboxylation in that order of the surface of the support.

### BEST MODES OF CARRYING OUT THE INVENTION

On the support for hybridization of the invention, a hybridized oligonucleotide, cDNA or gDNA is immobilized through chemical modification, and the support is characterized in that the oligonucleotide, cDNA or gDNA is immobilized thereon vertically to the support. Accordingly, the support makes it easy to read the cDNA information of monobasic polymorphism.

After the hybridized oligonucleotide has been immobilized on the support, the complementary oligonucleotide is dehybridized and then the information of the oligonucleotide having remained on the support is read.

To that effect, the oligonucleotide is inevitably double-stranded nucleic acid such as DNA, etc. The oligonucleotide includes natural ones that are derived from higher animals, fungi, bacteria, viruses, etc., as well as modified ones that are derived from the natural ones by artificially modifying their structures, and synthetic ones. Preferably, the oligonucleotide may be synthesized in a simplified manner according to the method mentioned below.

It is desirable that the number of the bases that constitute the oligonucleotide is from 10 to 50.

The support for hybridization of the invention may be fabricated in a simplified manner according to the method mentioned below.

### (1) Chemical modification of support for hybridization:

In the invention, the oligonucleotide as above is immobilized on a chemically-modified support for hybridization.

The support for hybridization includes glass, diamond; metals such as gold, silver, copper, aluminum, tungsten, molybdenum; laminates of the above-mentioned glass, diamond or metals with ceramics; and plastics such as polycarbonates, fluororesins.

Any other materials are usable so far as they are chemically stable, for example, graphite, diamond-like carbon, etc. Also usable are mixtures or laminates of the above-mentioned materials. For example, glass slides coated with diamond-like carbon are usable herein.

Of those, preferred is diamond or diamond-like carbon as the immobilization density of DNA thereon is extremely high.

The diamond may be any of synthetic diamond, high-pressure molded diamond, or natural diamond, etc. Its structure may have any form of single-crystal or polycrystal. From the viewpoint of productivity thereof, diamond is preferred that is produced through vapor-phase synthesis, for example, through microwave plasma-assisted CVD.

Diamond or diamond-like carbon may be formed in any known method. For example, the method includes microwave plasma-assisted CVD, ECRCVD, IPC, DC sputtering, ECR sputtering, ion plating, arc ion plating, EB vapor deposition, resistance heating vapor deposition, etc. Regarding metal powder, ceramic powder or the like, the material may be pressed into a green compact by the use of a pressing machine and it may be sintered at high temperatures.

Preferably, the surface of the support is intentionally roughened. The roughened surface is favorable for immobilizing a large quantity of DNA or the like, since its surface area increases. The shape of the support is not specifically defined, and may be tabular, yarn-like, spherical, polygonal, powdery, etc.

The support for hybridization that comprises diamond or diamond-like carbon may be a composite (for example, a two-phase composite) of diamond or diamond-like carbon with any other substance.

The chemical modification is for enabling polynucleotide bonding to the modified support, and is attained by substituting the surface of a solid support with a hydrocarbon group that has, at its terminal, a functional group such as a hydroxyl group, a carboxyl group, an epoxy group or an amino group. Preferably, the hydrocarbon part of the hydrocarbon group has from 0 to 12 carbon atoms, more preferably from 0 to 6 carbon atoms. Its examples are monocarboxylic acids such as formic acid, acetic acid, propionic acid; dicarboxylic acids such as oxalic acid, malonic acid, succinic acid, maleic acid, fumaric acid; and polycarboxylic acids such as trimellitic acid. Of those, oxalic acid and succinic acid are preferred.

Preferably, the hydrocarbon group is amido-bonded to the surface of the solid support. The amido-bonding facilitates and enhances the chemical modification.

The chemical modification may be attained by irradiating the surface of a support for hybridization with UV rays to chlorinate it, further irradiating it with UV rays in ammonia gas to aminate it, and thereafter carboxylating it with a suitable acid chloride or polycarboxylic acid anhydride.

### (2) Immobilization of hybridized oligonucleotide:

Next, a hybridized oligonucleotide (hereinafter referred to as oligonucleotide H) is immobilized in the chemically-modified part through amido-bonding. The support for hybridization is spotted with a liquid that contains the oligonucleotide H (see SEQ ID NO 5 in Sequence Listing) whereby the primary amine contained at the 5'-terminal of the oligonucleotide is bonded to the support. Before the immobilization, the hydrocarbon group terminal of chemical modification is preferably activated with a dehydrating condensation agent, as it facilitates the immobilization. In particular, carbodiimide is preferred for the dehydrating condensation agent.

Apart from the method, the surface of the support for oligonucleotide immobilization thereon may be chlorinated and aminated (but not carboxylated), and the primary amino group thus formed may be condensed through dehydration with one ester group of an activated diester that is prepared through pre-activation of a dicarboxylic acid with N-hydroxysuccinimide.

In consideration of the easiness in amido-bonding to the chemically-modified support for hybridization, it is desirable that the immobilization side has from 1 to 10 bases of a primary amine such as deoxyadenylic acid, deoxycytidylic acid or deoxyguanylic acid. Since the oligonucleotide H is immobilized on the support for hybridization in the direction vertical to the surface of the support, its DNA information is easy to read.

More preferably, it is desirable that from 1 to 20 bases on the immobilization side (5'-terminal) are deoxyadenylic acid, deoxycytidylic acid or deoxyguanylic acid.

### (3) The oligonucleotide H is dehybridized.

The support for hybridization on which the oligonucleotide has been immobilized is dipped in hot water at about 96°C and then washed with water for dehybridization.

### EXAMPLES

### Example 1 (Support for hybridization with EcoRI-cleaved gDNA immobilized thereon):

### (1) Chemical modification of support for hybridization:

Diamond chips were irradiated with UV rays in chlorine gas for their surface chlorination, and further irradiated with UV rays in ammonia gas for amination. Then, these were dipped in 1,4-dioxane containing 10 % by weight of an acid chloride for carboxylation to obtain chemically-modified chips.

### (2) Cleavage of λ phase DNA with EcoRI:

A λ phase DNA was cleaved with *Eco*RI to recover a 21-kbp fragment.

The chemically-modified chips obtained in (1) were activated and esterified with hydrogen cyanamide and N-hydroxysuccinimide, and dipped in an aqueous solution of the previously-obtained λ phage 21-kbp fragment (concentration, 1 µg/ µl) whereby the gDNA was immobilized on the support.

The amplification through PCR of the 500-bp fragment of that fragment was confirmed, and this supports the immobilization of the gDNA in the direction vertical to the chips.

### (3) Dehybridization of hybridized gDNA:

The chips obtained in (2) were dipped in hot water at 96°C and washed for dehybridization.

### (4) Cy3 labeling of λ phage 21-kbp fragment:

The 21-kbp λ phase obtained in (2) was Cy3-labeled by the use of Label IT™ (by PanVera), and formed into an aqueous solution (1 µg/µl). The solution was heated at 96°C for 5 minutes whereby the double-stranded structure was converted into a single-stranded structure. The chips dehybridized in (3) were dipped in the resulting solution for hybridization, and the fluorescence intensity thereof was measured with a fluorescence scanner. As a result, the 21-kbp λ phage was immobilized to a degree of 30 fmol/mm². Example 2 (Detection of monobasic mutation with oligonucleotide-immobilized hybridization support):

### (1) Chemical modification of support for hybridization:

A glass slide coated with soft diamond was irradiated with UV rays in chlorine gas to thereby chlorinate its surface, and then this was further irradiated with UV rays in ammonia gas to aminate it. Then, this was dipped in 1,4-dioxane containing 10 % by weight of an acid chloride for carboxylation to obtain chemically-modified chips.

### (2) Immobilization of oligonucleotide:

Using a spotter, oligonucleotide solutions A and B that had been prepared to have a concentration of 10 pmol/µl were immobilized on the support for hybridization obtained in (1). The oligonucleotide A (see SEQ ID NO 1 in Sequence Listing) was hybridized with oligonucleotide A' (see SEQ ID NO 2 in Sequence Listing), while the oligonucleotide B (see SEQ ID NO 3 in Sequence Listing) was with oligonucleotide B' (see SEQ ID NO 4 in Sequence Listing), both at 4°C for 5 hours, and then they were used as the samples.

### (3) Hybridization:

The oligonucleotide-immobilized hybridization support that had been obtained in (2) was put into hot water at 96°C, then the oligonucleotide solution A (not hybridized) having a concentration of 1 pmol/µl was put on it, and this was covered with a glass cover. With that, this was hybridized at 5°C for 12 hours.

### (4) Fluorescence observation:

The support obtained in (3) was washed and dried, and observed with a fluorescence scanner. As a result, the spot of oligonucleotide A immobilization gave fluorescence, but the spot of oligonucleotide B immobilization did not.

### INDUSTRIAL APPLICABILITY

On the support for hybridization of the present invention, a hybridized oligonucleotide is immobilized in the direction vertical to the support. Therefore, the support enables easy and accurate reading of the information of DNA, etc. Before reading, the support is dehybridized and therefore gives more accurate information.

## Claims

1. A support for hybridization, which is **characterized in that** a hybridized oligonucleotide, cDNA or gDNA is immobilized thereon.

2. The support for hybridization as claimed in claim 1, wherein the hybridized oligonucleotide, cDNA or gDNA contains at least two continuous, primary amine-having nucleotides.

3. The support for hybridization as claimed in claim 1 or 2, wherein the primary amine-having nucleotide is deoxyadenylic acid, deoxycytidylic acid or deoxyguanylic acid.

4. The support for hybridization as claimed in claims 1 to 3, wherein the primary amine-having nucleotides exist on the side of the 5'-terminal.

5. The support for hybridization as claimed in claims 1 to 4, wherein the oligonucleotide has a 5'-overhang terminal.

6. The support for hybridization as claimed in claim 1 or 2, wherein the cDNA is synthesized by the use of an oligonucleotide, as a primer, which contains at least two continuous, primary amine-having nucleotides on the side of the 5'-terminal.

7. The support for hybridization as claimed in claim 1 or 2, wherein the gDNA is **characterized in that** it is cleaved with a restriction enzyme so as to contain at least two continuous, primary amine-having nucleotides on the side of the 5'-terminal.

8. The support for hybridization as claimed in claim 1, 2 or 7, wherein the gDNA is **characterized in that** it is cleaved with a restriction enzyme so as to contain at least two continuous, primary amine-having nucleotides on the side of the 5'-overhang terminal.

9. The support for hybridization as claimed in claims 1 to 8, wherein deoxyadenylic acid, deoxycytidylic acid or deoxyguanylic acid is immobilized on the side of the support for hybridization.

10. The support for hybridization as claimed in claim 9, wherein the hybridized oligonucleotide, cDNA or gDNA that is immobilized on the support for hybridization has from 1 to 10 primary amine-having nucleotides at the terminal for immobilization.

11. A method for immobilizing hybrid, which comprises chemically modifying a support for hybridization, immobilizing a hybridized oligonucleotide, cDNA or gDNA on it, and then dehybridizing the complementary oligonucleotide, cDNA or gDNA not immobilized on the support.

12. The method for immobilizing hybrid as claimed in claim 11, wherein the chemical modification of the support for hybridization comprises chlorination, amination and carboxylation of the surface of the support.


SEQUENCE LISTING

<110> Toyo Kohan Co., Ltd.

<120> Supports for hybridization and method of immobilizing hybrid

<130>H 1774 EP

<150>JP 2000-344651

<151>2000-11-13

<160>5

<210>1

<211>27

<212>DNA

<213>artificial sequence

<400>1

aaa aaa aaa agt ccg gct cag cta gtc 27

<210>2

<211>18

<212>DNA

<213>artificial sequence

<400>2

gac tag ctg agc cgg act 18

<210>3

<211>27

<212>DNA

<213>artificial sequence

<400>3

aaa aaa aaa agt ccg tct cag cta gtc 27

<210>4

<211>18

<212>DNA

<213>artificial sequence

<400>4

gac tag ctg aga cgg act 18

<210>5

<211>27

<212>DNA

<213>artificial sequence

<400>5

aaa aaa aaa agt tca tct cag gta ctc 27